# EUROPÄISCHE PATENTSCHRIFT

(11) **EP 0 671 973 B1**
(45) Veröffentlichungstag und Bekanntmachung des Hinweises auf die Patenterteilung: **23.10.1996**
(21) Anmeldenummer: 94928847.6
(22) Anmeldetag: 01.10.1994
(51) Int. Cl.: B01F 17/00, C11D 3/38, C12N 1/00

(54) **GRENZFLÄCHENCHEMISCH AKTIVE VERBINDUNGEN AUS NACHWACHSENDEN ROHSTOFFEN**
SURFACE ACTIVE COMPOUNDS PRODUCED FROM REGENERATIVE STARTING MATERIALS
COMPOSES A ACTIVITE CHIMIQUE INTERFACIALE ISSUS DE PRODUITS DE DEPART A POST-CROISSANCE

(30) Priorität: 05.10.1993 DE 4334689
(43) Veröffentlichungstag der Anmeldung: 20.09.1995
(73) Patentinhaber: Olschewski, Brigitte, Dr., 12557 Berlin (DE); Olschewski, Max, 12557 Berlin (DE)
(72) Erfinder: Olschewski, Brigitte, Dr., 12557 Berlin (DE); Olschewski, Max, 12557 Berlin (DE)
(86) Internationale Anmeldenummer: EP9403263
(87) Internationale Veröffentlichungsnummer: WO9509691

(56) Entgegenhaltungen:
- EP-A- 0 220 676
- EP-A- 0 379 101
- EP-A- 0 417 619
- WO-A-91/19777
- WO-A-92/13866
- CHEMICAL ABSTRACTS, vol. 113, no. 16, 15. Oktober 1990, Columbus, Ohio, US; abstract no. 138306n, Seite 392 ;

## Beschreibung

Die Erfindung betrifft eine Mischung grenzflächenchemisch aktiver Verbindungen, die aus mikrobiellen Biomassen sowie Fetten einerseits und die Kettenlänge sowie den HLB-Wert der Einzelbestandteile der Mischung regulierender niedermolekularer organischer Verbindungen andererseits erhalten werden.

Diese Mischungen grenzflächenchemisch aktiver Verbindungen sind als im wesentlichen nichtionische Tenside mit anionischem Anteil beispielsweise als Reinigungs- oder Schäumungsmittel sowie als Emulgatoren für die unterschiedlichsten organischen Stoffe in Wasser, d.h. für Öl-in-Wasser-Emulsionen und darüber hinaus im weitesten Sinne als Netzmittel einsetzbar.

Bisher sind vor allem die folgenden, hochwirksamen Tenside in hoher Produktmenge auf den Markt gekommen: Alkylsulfonate, Alkylbenzolsulfonate, Alkylethersulfate, Alkoholethoxylate, Alkylsulfate und Seife. Einige von ihnen sollten wegen ihrer mangelnden biologischen Abbaubarkeit durch ökologisch verträglichere Produkte ersetzt werden.

Im Zeitalter eines steigenden Umweltbewußtseins liegt der Schwerpunkt der Tensidchemie heute auf der Synthese von umweltverträglichen Tensiden auf der Basis nachwachsender Rohstoffe (B.Fabry, Chemie in unserer Zeit 25 (1991) Nr.4). Dabei zeichnen sich hinsichtlich eines guten Eigenschaftsbildes und eines günstigen ökologischen Vergleiches zwei wesentliche Tensidklassen ab, nämlich die Alkylpolyglucoside und die Eiweiß-Fettsäure-Kondensationsprodukte.

Zur Herstellung von Polyglucosiden werden entweder Saccharidhydroxylgruppen mit Fettalkoholen sauer veräthert (DE-40 06 192 und EP-448 799), oder Saccharide mit Fettsäuren verestert (EP-268 974) bzw. darüber hinaus noch oligoalkoxyliert (DE-41 03 681 und EP-405 967) oder Aldehydgruppen der Saccharide durch reduzierende Aminierung und anschließende Reaktion der entstandenen Aminogruppen mit Säurechlorid zu Amiden umgesetzt (DE-35 38 451). Außerdem zeigen Zusätze solcher Polyglucoside zu Alkylethersulfat oder Alkylsulfonat synergistische Effekte (DE-40 19 790). Alkylpolyglucoside besitzen interessante anwendungstechnische Eigenschaften wie synergistische Wechselwirkungen mit vielen anderen Tensiden, höhere Schaumaktivität als übliche nichtionische Tenside, verbesserte Hautverträglichkeit und gute biologische Abbaubarkeit.

Eine weitere Gruppe der milden Tenside mit guter Haut- und Schleimhautverträglichkeit sind die Eiweiß-Fettsäure-Kondensationsprodukte. Diese werden mit den normalen wasch- und reinigungsaktiven Mitteln kombiniert, um die Hautverträglichkeit der Alkylsulfate, Olefinsulfonate und Alkylbetaine zu verbessern. Dazu werden beispielsweise Eiweißhydrolysate der Molmasse 3000 bis 7000 mit Säurechlorid (EP-417 619 und EP-379 101) oder auch mit Fettsäureestern (DD-273 275) kondensiert. Die Eiweiß-Fettsäure-Kondensate werden immer in Kombination mit anderen Tensiden eingesetzt. Die Oberflächenspannung ihrer wässrigen Lösung beträgt auch bei höheren Konzentrationen 30 mN/m. Die zu ihrer Herstellung dienenden Eiweißhydrolysate sind aus pflanzlichen und tierischen Proteinen erhältlich.

Die vorteilhaften Eigenschaften dieser Produkte bestehen in der fehlenden Toxizität, ihrer zeithemmenden Wirkung in Kombination mit üblichen Tensiden und in einer guten Kompatibilität mit anionischen, nichtionischen sowie teilweise mit quarternären Ammoniumverbindungen. Sie selbst besitzen eine milde Reinigungswirkung.

Weiterhin werden Mono- und Diglyceride, d.h. Umesterungsprodukte von Fetten mit Fettsäuren, in der Lebensmittelindustrie als Emulgatoren eingesetzt (DE-27 05 608, DE-19 37 433, DE-31 36 743, EP-305 901, EP-289 968).

Desweiteren wurde in zwei neueren Patentanmeldungen die Herstellung von Tensiden auf der Basis von mikrobiellen Biomassen vorgeschlagen. Diese Produkte ergeben allerdings ein relativ unspezifisches und lediglich im lipophilen Molekülteil variables Eigenschaftsbild. Es handelt sich hierbei einmal um die chemische Umsetzung von Hefe mit Säurehalogeniden, Alkylhalogeniden und Ethylenoxid zu grenzflächenaktiven Stoffen mit niedriger Oberflächenspannung (DE-A-43 04 550) sowie die chemische Umsetzung von mikrobiellen Biomassen mit nativen Fetten, ebenfalls zu Produkten mit guten grenzflächenchemischen Eigenschaften (DE-C-43 07 270). In beiden Herstellungsvarianten ergeben sich bioabbaubare Tenside mit niedriger Oberflächenspannung ihrer wässrigen Lösungen, wobei allerdings die HLB bei Werten von maximal 10 oder deutlich darunter liegt; d.h. es handelt sich hierbei im wesentlichen um Tenside des Wasser-in-Öl Typs. Außerdem bewegen sich die Molmassen der Anteile dieser Produktgemische eher im höheren oligomeren Bereich, so daß infolge dessen die Wasserlöslichkeit der Produkte, insbesondere bei den Varianten mit Fetten der längeren Kettenlänge, nicht optimal sein dürfte.

Die Erfindung hat den Zweck der Herstellung grenzflächenaktiver Verbindungen mit einer Oberflächenspannung ihrer wässrigen Lösung von < 29 mN/m, die damit in besonderer Weise zur Benetzung von unterschiedlichen, insbesondere organischen Stoffen mit polarem und unpolarem sowie aliphatischem und aromatischem Charakter geeignet sind.

Es bestand dabei die Aufgabe, mikrobielle Biomassen, die zahlreiche und unterschiedliche reaktionsfähige Gruppen enthalten, im Gemisch mit natürlichen Fetten und/oder Ölen, die ebenfalls unter-schiedliche reaktionsfähige Gruppen enthalten, durch chemische Umsetzung mit niedermolekularen organischen Verbindungen, die in der Lage sind, die Kettenlänge sowie den HLB- Wert der Einzelbestandteile der resultierenden Mischung zu regulieren, in grenzflächenaktive Stoffe zu überführen.

Erfindungsgemäß wird aus dem Gemisch einer mikrobiellen Biomasse mit natürlichen, pflanzlichen und/oder tierischen Fetten und/oder Ölen sowie deren Mischungen durch chemische Umsetzung mit geeigneten niedermolekularen organischen Verbindungen in wässrigem Medium und in Anwesenheit eines katalytisch wirksamen Mittels eine Mischung grenzflächenchemisch aktiver Verbindungen hergestellt, wobei die reaktionsfähigen Gruppen aller Reaktanden, d.h. sowohl diejenigen als Bestandteil einer Molekülkette als auch die außenständigen funktionellen Gruppen, miteinander in der Weise reagieren, daß durch dabei anteilig eintretende Kettenspaltungen einerseits und neu oder andersartig erzeugte funktionelle Gruppen andererseits sich sowohl eine gewünschte mittlere Kettenlänge als auch eine gewünschte HLB des resultierenden grenzflächenaktiven Stoffes einstellen läßt.

Geeignete mikrobielle Biomassen sind beispielsweise Hefen wie Bäckerhefe, Brauhefe, Gärhefe in frischem Zustand wie auch als Abfallbiomasse oder Bakterien, beispielsweise in Form von Klärschlamm oder einzelligen Algen.

Die einzusetzenden nativen Fette und/oder Öle, auch Abfallfette bzw. -öle, können ohne jede Vorbehandlung verwendet werden. Erfindungsgemäß eingesetzte niedermolekulare organische Verbindungen sind monomere bis oligomere, monofunktionelle bis hexafunktionelle Stoffe, deren funktionelle Gruppen primäre oder sekundäre Hydroxylgruppen, Amino- oder Iminogruppen, Carboxyl-, Sulfonyl-, Phosphonyl- oder Phosphorsäuregruppen sein können, bzw. derartige unterschiedliche Gruppen im selben Molekül wie beispielsweise Oxycarbonsäuren oder monomere bis oligomere Aminosäuren tragen können, bzw. Mischungen daraus.

Andersartig erzeugte funktionelle Gruppen zum Zwecke der gezielten HLB-Einstellung können durch Umsetzung der vorhandenen H-aciden Gruppen mit niederen Epoxiden erhalten werden.

Das molare Verhältnis der reaktionsfähigen Gruppen für die erfindungsgemäße Umsetzung soll in der Summe aus Biomasse und Fett bzw. Öl einerseits zu niedermolekularer organischer Verbindung andererseits wie 10:1 bis 1:2 sein, bevorzugt 3:1 bis 1:1.

Die für die Umsetzung notwendigen katalytisch wirksamen Mittel können beispielsweise Alkalimetallhydroxide sein.

Als besonders vorteilhaft ist die Tatsache hervorzuheben, daß die Reaktion überraschenderweise lediglich im wässrigen Medium durchführbar ist und demzufolge ohne irgendwelche Lösungsmittel auskommt.

Als ebenfalls besonders vorteilhaft ist die Tatsache anzusehen, daß im Falle des erfindungsgemäßen Einsatzes von säuregruppenenthaltenden niedermolekularen organischen Verbindungen die resultierenden grenzflächenchemisch aktiven Stoffe in wässriger Lösung in der Lage sind, mehrwertige Kationen in Form von Salzen anzulagern.

Die erfindungsgemäß erhaltenen Produkte weisen eine ausgezeichnete biologische Abbaubarkeit auf.

Die Erfindung wird nachstehend ohne einschränkenden Charakter an Ausführungsbeispielen näher erläutert:

### Beispiel 1:

400 g Brauhefe (30%-ig) werden mit 150 g 40%-iger Natronlauge und mit 40 g Polyethylenglykol der Molmasse 300 versetzt und 30 Minuten lang bei einer Temperatur von 80° C gerührt. Dann werden 50 g Kokosfett und 75 g Kokosöl hinzugegeben und das Ganze für weitere 3 Stunden bei einer Temperatur von 75° C gerührt.
Nach dem Abkühlen erhält man ein etwa 43%-iges Batch von bräunlicher Farbe, mit einem pH-Wert von 10,5.

### Beispiel 1 A:

400 g Brauhefe (30%-ig) werden mit 150 g 40%-iger Natronlauge und mit 40 g Polyethylenglykol der Molmasse 300 versetzt und 30 Minuten lang bei einer Temperatur von 80°C gerührt. Dann werden 50 g Kokosfett und 75 g Kokosöl hinzugegeben und das Ganze für weitere 3 Stunden bei einer Temperatur von 75°C gerührt. Anschließend wird das Reaktionsgemisch bei der selben Temperatur innerhalb 30 Minuten mit 58 g Propylenoxid versetzt und 1 Stunde nachgerührt.
Nach dem Abkühlen erhält man ein etwa 47%-iges Batch von bräunlicher Farbe, mit einem pH-Wert von 10.

### Beispiel 2:

400 g Brauhefe (30%-ig) werden mit 150 g 40%-iger Natronlauge und mit 30 g Polypropylenglykol der Molmasse 400 versetzt und 30 Minuten lang bei einer Temperatur von 80°C gerührt. Dann wird ein Gemisch aus 70 g Rindertalg und 80 g Palmöl hinzugegeben und das Ganze für weitere 4 Stunden bei einer Temperatur von 80°C gerührt.
Nach dem Abkühlen erhält man ein etwa 51%-iges Batch von bräunlicher Farbe, mit einem pH-Wert von 10.

### Beispiel 3:

400 g Bäckerhefe (25%-ig) werden mit 120 g 40%-iger Natronlauge sowie mit 20 g Citronensäure und 10 g Sorbit versetzt und 30 Minuten lang bei einer Temperatur von 80°C gerührt. Dann wird ein Gemisch aus 100 g Milchfett und 20 g gehärtetem Pflanzenfett (C16) hinzugegeben und das Ganze für weitere 3 Stunden bei einer Temperatur von 70°C gerührt.
Nach dem Abkühlen erhält man ein etwa 47%-iges Batch von bräunlicher Farbe, mit einem pH-Wert von 9,5.

### Beispiel 4:

1000 g Belebtschlamm (25%-ig) werden mit 300 g 40%-iger Natronlauge sowie mit 15 g Citronensäure, 15 g Maleinsäure, 40 g Polyethylenglykol der Molmasse 600 und 20 g Polyethylenglykol der Molmasse 200 versetzt und 1 Stunde lang bei einer Temperatur von 80°C gerührt. Dann wird ein Gemisch aus 150 g Sonnenblumenöl und 100 g Rapsöl hinzugegeben und das Ganze für weitere 5 Stunden bei einer Temperatur von 75°C gerührt.
Nach dem Abkühlen erhält man ein etwa 43%-iges Batch von braungräulicher Farbe, mit einem pH-Wert von 9,5.

### Beispiel 5:

1000 g Belebtschlamm (35%-ig) werden mit 500 g 40%-iger Natronlauge sowie mit 10 g Butanol, 10 g Butandiol, 40 g Polyethylenglykol der Molmasse 400, 10 g Polyethylenglykol der Molmasse 1000 und 50 g Maleinsäure versetzt und 1 Stunde lang bei einer Temperatur von 80°C gerührt. Dann werden 250 g Geflügelabfallfett hinzugegeben und das Ganze für weitere 3 Stunden bei einer Temperatur von 75°C gerührt.
Nach dem Abkühlen erhält man ein etwa 50%-iges Batch von braungräulicher Farbe, mit einem pH-Wert von 10,5.

### Beispiel 6:

400 g Faulschlamm (90%-ig TS) werden mit 600 g Wasser, 150 g 40%-iger Natronlauge sowie mit 40 g Polyethylenglykol der Molmasse 300 versetzt und 30 Minuten lang bei einer Temperatur von 80°C gerührt. Dann werden 50 g Kokosfett und 75 g Kokosöl hinzugegeben und das Ganze für weitere 3 Stunden bei einer Temperatur von 75°C gerührt. Anschließend wird das Reaktionsgemisch bei der selben Temperatur innerhalb 40 Minuten mit 44 g Ethylenoxid versetzt (Kühlung mit Trockeneis/ Methanol) und 1 Stunde nachgerührt.
Nach dem Abkühlen erhält man ein etwa 60%-iges Batch von brauner Farbe, mit einem pH-Wert von 10.

Die Tensideigenschaften der erhaltenen Produkte sind in der nachfolgenden Tabelle aufgeführt. Die Oberflächenspannung wurde bei pH 7 gemessen.

**Tabelle**

| Produkt von Beispiel | Oberflächenspannung mN/m | CMC g/l | HLB |
|---|---|---|---|
| 1 | 22,5 | 1 x 10° | 12,0 |
| 1 A | 22,1 | 4 x 10⁻¹ | 10,5 |
| 2 | 20,3 | 6 x 10⁻¹ | 7,3 |
| 3 | 23,3 | 2 x 10⁻¹ | 14,5 |
| 4 | 26,0 | 3 x 10⁻¹ | 15,0 |
| 5 | 23,8 | 1,5 x 10⁻¹ | 13,2 |
| 6 | 23,7 | 4 x 10⁻¹ | 13,7 |

## Patentansprüche

1. Mischung grenzflächenchemisch aktiver Verbindungen aus nachwachsenden Rohstoffen mit zwischen 6 und 16 einstellbarer HLB, **dadurch gekennzeichnet**, daß sie durch Reaktion von Hefen, Bakterien oder einzelligen Algen sowie natürlichen pflanzlichen und/oder tierischen Fetten und/oder Ölen durch chemische Umsetzung mit mono- bis hexafunktionellen monomeren bis oligomeren organischen Verbindungen, deren funktionelle Gruppen primäre und/oder sekundäre Hydroxylgruppen, Amino- und/oder Iminogruppen, bzw. Carboxyl-, Sulfonyl-, Phosphonyl- oder Phosphorsäuregruppen sowie Epoxidgruppen sind bzw. derartige unterschiedliche Gruppen im selben Molekül tragen bzw. Mischungen daraus sind, erhalten werden, wobei das molare Verhältnis der Summe aller reaktionsfähigen Gruppen der mikrobiellen Biomasse und der Fette bzw. Öle, d.h. sowohl derjenigen innerhalb der Molekülketten als auch der außenständigen, im Verhältnis zu denjenigen der monomeren bis oligomeren organischen Verbindungen sich wie 10:1 bis 1:2, bevorzugt 3:1 bis 1:1, verhält, und wobei die Reaktion im wässrigen Medium in Gegenwart von Alkalimetallhydroxiden als katalytisch wirksamem Mittel durchgeführt wird.

2. Mischung nach Anspruch 1, **dadurch gekennzeichnet**, daß aus der Umsetzung resultierende außenständige Säurerestgruppen mehrwertige Metallionen salzartig anlagern.

3. Verwendung der Mischungen gemäß Anspruch 1 und 2 für Reinigungszwecke im allgemeinen sowie als Emulgatoren.

## Claims

1. Mixture of chemically surface-active compounds from HLB adjustable between 6 and 16 according to growing raw materials, **characterised by the fact** that they are obtained by means of reaction of yeasts, bacteria or unicellular algae as well as naturally occurring vegetable and/or animal fats and/or oils by means of chemical conversion with monofunctional to hexafunctional monomeric to oligomeric organic compounds whose functional groups are primary and/or secondary hydroxyl groups, amino and/or imino groups or carboxyl, sulphonyl, phosphonyl or phosphoric-acid groups as well as epoxide groups or carry such different groups in the same molecule, or are mixtures of these, the molar ratio of the sum of all reactive groups of the microbial organic substances and of the fats or oils, i.e. both of those within the molecular chains and of the external ones, being 10:1 to 1:2, preferably 3:1 to 1:1, and the reaction being carried out in a watery medium in the presence of alkali metal hydroxides as catalytically effective agents.

2. Mixture according to Claim 1, **chacterised by the fact** that external acid-residue groups resulting from the conversion attach polyvalent metal ions like salts.

3. The above mixtures in accordance with Claims 1 and 2 are to be used for purification purposes in general and as emulsifying agents in particular.

## Revendications

1. Mélange de combinaisons tensio-actives de HLB ajustable suivant des matières premières intensifiantes entre 6 et 16, **caractérisé par le fait** que celles-ci sont obtenues par une réaction de levures, de bactéries ou d'algues unicellulaires ainsi que de matières grasses et/ou d'huiles végétales et/ou animales naturelles moyennant une conversion chimique à l'aide de composés organiques monofonctionnels à hexafonctionnels, monomères à oligomères dont les groupes fonctionnels sont des groupes primaires et/ou secondaires hydroxyles, amino et/ou imino ou carboxyles, sulfonyles, phosphonyles ou d'acide phosphorique ainsi que des groupes époxides ou qui portent de tels groupes différents dans la même molécule ou qui en Sont les mélanges, le rapport molaire de la somme de tous les groupes réactifs des substances organiques microbielles et des matières grasses ou des huiles, c'est-à-dire, tant des groupes à l'intérieur des chaînes moléculaires que des groupes à l'extérieur, par rapport à ceux des combinaisons organiques monomères à oligomères, allant de 10:1 à 1:2, de préférence de 3:1 à 1:1, et la réaction s'effectuant dans un milieu aqueux en présence d'hydroxides de métaux alcalins agissant comme agents catalytiques.

2. Mélange conformément à la revendication no. 1, **caractérisé par le fait** que des groupes extérieurs d'acides restants résultant de la conversion attachent, de manière saliforme, des ions polyvalents de métaux.

3. Ces mélanges, conformément aux revendications no. 1 et 2, sont destinés à des fins de purification en général et doivent servir, en particulier, d'émulsifiants.
